# EUROPÄISCHE PATENTANMELDUNG

(11) **EP 1 616 490 A1**
(43) Veröffentlichungstag der Anmeldung: **18.01.2006**
(21) Anmeldenummer: 05105901.2
(22) Anmeldetag: 30.06.2005
(51) Int. Cl.: A23L 1/30, A23L 1/302, A23L 1/303, A23L 1/304, A61K 31/07, A61K 31/355, A61K 31/375, A61K 31/455, A61K 33/30, A61K 33/34, A61P 27/02

(54) **Zusammensetzung enthaltend Lutein sowie Zink und Kupfer**

(30) Priorität: 30.06.2004 DE 202004010212 U
(71) Anmelder: Dr. GERHARD MANN chem.-pharm. Fabrik GmbH, D-13581 Berlin (DE)
(72) Erfinder: Bellmann, Günther, Dr., 13593, Berlin (DE); Claus-Herz, Gudrun, Dr., 14167, Berlin (DE)
(74) Vertreter: Michalski, Stefan

(57) **Zusammenfassung**

Die Erfindung betrifft eine Lutein-enthaltende Zusammensetzung, wobei die Zusammensetzung Lutein sowie Zink- und Kupferverbindungen, vorzugsweise in Form von Zinkgluconat und Kupfer(II)-gluconat, umfasst.

## Beschreibung

Die vorliegende Erfindung betrifft eine Lutein-enthaltende Zusammensetzung. Die Lutein-enthaltende Zusammensetzung kann unter anderem als Nahrungsergänzungsmittel oder zur ergänzenden bilanzierten Diät beispielsweise bei altersbedingter Makuladegeneration verwendet werden.

Es ist allgemein bekannt, dass das Auge durch den Sehprozess hohen Belastungen ausgesetzt ist. Durch Licht- und Sauerstoffeinwirkung entstehen sogenannte "freie Radikale", die zu Schädigungen oder krankhaften Veränderungen in der Netzhaut führen können.

Es ist weiterhin allgemein bekannt, dass äußere Einflüsse, wie unausgewogene Ernährung, Nikotin und Alkohol, Umweltverschmutzung, Stress oder UV-Strahlung, zu einem vermehrten Auftreten von Radikalen im Organismus führen können. Aber auch die normalen Stoffwechselvorgänge setzen kurzfristig hochaktive Substanzen frei, die den Körper belasten und schädigen können. Zum Abbau dieser "freien Radikale" besitzt der Körper ein eigenes "antioxidatives" Schutzsystem, an dem die Vitamine C und E, das vitaminähnliche Carotinoid Lutein sowie das Spurenelement Zink entscheidend beteiligt sind. Einen optimalen Schutz bietet das System nur dann, wenn alle seine aktiven Bestandteile in ausreichender Menge vorhanden sind.

Das Auge ist durch den Sehprozess und die ständige Lichteinwirkung in erhöhtem Maß oxidativem Stress ausgesetzt. Es hat daher einen erhöhten Bedarf an einer ausreichenden Versorgung mit antioxidativen Stoffen wie Vitamin C, Vitamin E, Zink und Lutein. Zu den Organen, welche eine besonders hohe Konzentrationen des Carotinoids Lutein benötigen, zählt die Makula des Auges. Unter dem medizinischen Begriff "Makula lutea" ist die Netzhautmitte, also der Punkt des schärfsten Sehens zu verstehen. Hier befinden sich die empfindlichsten Sehzellen des Auges.

Bei einer unzureichenden Versorgung der Makula mit antioxidativen Stoffen kann das Risiko der Entwicklung einer "altersbedingten Makuladegeneration" (AMD) ansteigen. Risikofaktoren für die Entstehung einer AMD sind beispielsweise eine unausgewogene Ernährung, Nikotingenuss, Sonnen-Strahlung, fortgeschrittenes Alter, oder blaue Augen. Die AMD hat ein Schwinden der Sehkraft zur Folge, im schlimmsten Fall bis hin zur Erblindung.

Im Stand der Technik bekannte Vitaminpräparate enthalten verschiedene, häufig hochdosierte, Zusammensetzungen an antioxidativ wirkenden Vitaminen und Spurenelementen. Ein Nachteil dieser Präparate ist ihre unzureichende Versorgung der Makula, die eine zentrale Funktion beim Sehprozess einnimmt.

Im Stand der Technik bekannte Zinkpräparate sind ebenfalls häufig hochdosierte Präparate, die Zinksulfat oder Zinkoxid enthalten. Diese Präparate haben jedoch den Nachteil, dass sie für eine Dauertherapie beziehungsweise eine zeitlich unbegrenzte Prophylaxe nicht geeignet sind, da die Dosis dieser Präparate zu Überdosierungen und gesundheitsschädlichen Nebenwirkungen führen können. Eine langfristige höher dosierte (> 25 mg Zink/Tag) Zinkeinnahme kann darüber hinaus zu einem Kupfermangel führen, der durch anämische Zustände gekennzeichnet sein kann. Eine Reduktion der alimentären Zinkzufuhr kann andererseits rasch zu einem Zinkmangel führen.

Ein weiterer Nachteil handelsüblicher antioxidativer Präparate besteht darin, dass die Altersabhängigkeit des Vitamin- und Mineralstoffbedarfs zu wenig berücksichtigt wird. Besonders im Alter können sich Mangelerscheinungen aufgrund von Mangel- oder Fehlernährung oder Krankheit rasch bemerkbar machen, insbesondere im Hinblick auf Erkrankungen wie AMD, denen durch darauf abgestimmte Präparate zu wenig Rechnung getragen wird.

Es besteht daher Bedarf an einem Mittel, das einer unzureichenden Versorgung der Makula mit Vitaminen und Spurenelementen entgegenwirkt. Darüber hinaus besteht Bedarf an einem Mittel, das als solches keine gesundheitsschädliche Nebenwirkung hervorruft und sowohl unbedenklich zur Präventionen von Mangelerscheinungen dienen kann, wie auch gegebenenfalls bei bereits bestehender altersbedingter Makuladegeneration verwendbar ist.

Es ist daher die Aufgabe der vorliegenden Erfindung, ein Mittel zur Verfügung zu stellen, das wenigstens einen der vorgenannten Nachteile des Standes der Technik überwindet. Insbesondere ist es die Aufgabe der vorliegenden Erfindung, ein Mittel zur Verfügung zu stellen, das beispielsweise eine umfassende Versorgung der Makula mit Antioxidanzien bietet.

Diese Aufgabe wird durch eine Lutein-enthaltende Zusammensetzung gelöst, wobei die Zusammensetzung Lutein sowie Zink- und Kupferverbindungen, vorzugsweise in Form von Zinkgluconat und Kupfer(II)-gluconat, umfasst.

Es hat sich überraschend gezeigt, dass die Makulapigmentdichte durch Verabreichen des vitaminähnlichen Carotinoids Lutein deutlich gestärkt wurde. Es hat sich weiterhin überraschend gezeigt, dass das erfindungsgemäß optimiert verwendete vitaminähnliche Carotinoid einen verbesserten vorbeugenden Schutz der Makula bewirken kann.

Ohne auf eine bestimmte Theorie festgelegt zu sein wird angenommen, dass sich das vitaminähnliche Carotinoid Lutein in hohen Konzentrationen in der Makula selektiv anreichern und so diesen Bereich durch das Filtern des Lichts und das Abfangen der schädlichen "freien Radikale" schützen kann.

Weiterhin weist die erfindungsgemäße Zusammensetzung Zink- und Kupferverbindungen, vorzugsweise in Form von Zink- und Kupfersalzen auf. Bevorzugt weist die Zusammensetzung wasserlösliche und gut verträgliche Zink- und Kupfersalze auf, besonders bevorzugt weist die erfindungsgemäße Zusammensetzung Zink- und/oder Kupferverbindungen in Form von Zinkgluconat und/oder Kupfer(II)-gluconat auf. Es wird vermutet, dass zumindest das Spurenelement Zink an den antioxidativen Prozessen, die das Auge und insbesondere die Makula vor einer Schädigung durch Radikale schützen, beteiligt ist. Es hat sich überraschend gezeigt, dass durch eine Verwendung von Zinkgluconat die Bioverfügbarkeit des Zinks gegenüber einer Verwendung von anderen Zinksalzen wie Zinksulfat oder Zinkoxid verbessert war. Es wird vermutet, dass insbesondere die Versorgung der Makula mit Zink durch die Verwendung von Zinkgluconat verbessert wird.

Das erfindungsgemäß verwendete Zinkgluconat zeigt beispielsweise eine bessere Resorption als das häufig verwendete Zinksulfat. Insbesondere zeigt Zinkgluconat eine deutlich bessere Resorption als das auch häufig verwendete Zinkoxid. Es ist denkbar, dass durch die Verwendung des gut resorbierbaren Zinkgluconats der notwendige Bedarf an Zink durch Verabreichen einer geringeren Menge dieser Zinkverbindung als Tagesdosis bzw. Einzeldosis im Vergleich zu Zinksalzen wie Zinksulfat oder Zinkoxid gedeckt werden kann, und damit eine mögliche Überdosierung und die Gefahr von Nebenwirkungen sicher vermieden werden kann.

Weiterhin weist die Lutein-enthaltende Zusammensetzung Kupferverbindungen vorzugsweise in Form von Kupfer(II)-gluconat, auf, welches einem möglichen Kupfermangel, der aus der Einnahme von Zink resultieren kann, entgegenwirken kann. Vorteilhaft ist, dass die erfindungsgemäße Zusammensetzung aufgrund der Verwendung von Zinkgluconat einen äußerst gering dosierten Anteil an Kupferverbindungen umfasst.

Die Kombination der Spurenelemente Zink und Kupfer mit dem vitaminähnlichen Carotinoid Lutein stellt eine Zusammensetzung zur Verfügung, die in vorteilhafter Weise beispielsweise eine ausgewogene Versorgung der Makula mit antioxidativ wirkenden Mikronährstoffen bereit stellt. Die gezielte Zufuhr dieser Stoffe kann vorteilhafter Weise dazu beitragen, das Auge vor der Entstehung einer altersbedingten Makuladegeneration zu schützen oder deren Fortschreiten zu verlangsamen. Weiterhin wird durch die aufeinander abgestimmte Gabe der Spurenelemente Zink und Kupfer mit Lutein eine Überdosierung sicher vermieden und eine ausgewogene Versorgung zur Verfügung gestellt.

Die Lutein-enthaltende Zusammensetzung kann beispielsweise als Nahrungsergänzungsmittel oder als Mittel zur ergänzenden bilanzierten Diät oder als Mittel zur begleitenden Verabreichung, beispielsweise bei der Behandlung einer altersbedingten Makuladegeneration verwendet werden.

In vorteilhaften Ausführungsformen weist die Lutein-enthaltende Zusammensetzung weiterhin wenigstens ein Vitamin, vorzugsweise ausgewählt aus der Gruppe umfassend Vitamin C, Vitamin E und/oder Niacinamid, auf.

Eine Ergänzung der Lutein-enthaltenden Zusammensetzung mit Vitaminen ausgewählt aus der Gruppe umfassend Vitamin C, Vitamin E und/oder Niacinamid kann einen weiteren Vorteil der Zusammensetzung durch eine Verbesserung der antioxidativen Eigenschaften der Zusammensetzung bewirken. Eine Ergänzung der Zusammensetzung mit den antioxidativ wirkenden Vitaminen Vitamin C und/oder Vitamin E kann einen verbesserten Schutz des Auges und insbesondere der Makula durch eine Erhöhung des Anteils der durch diese Antioxidantien abgefangenen Radikale bewirken.

Eine umfassende Unterstüzung der Makula kann hierbei vermutlich durch Niacinamid bewirkt werden.

Ein weiterer Vorteil der Lutein-enthaltenden Zusammensetzung wird durch die antioxidativen Eigenschaften von Niacinamid verwirklicht, das verträglicher ist als die freie Säure Niacin. Die erhöhte Verträglichkeit der Lutein-enthaltenden Zusammensetzung kann sich hierbei insbesondere bei einer langfristigen Verwendung der Zusammensetzung positiv auswirken. Einen großen Vorteil kann die Verwendung von Niacinamid insbesondere bei älteren Menschen bewirken, die allgemein empfindlicher auf Unverträglichkeiten reagieren.

Mittels der erfindungsgemäßen Zusammensetzung kann ein umfassender Schutz vor Radikalen zur Verfügung gestellt werden, der eine Schädigung des Auges und insbesondere der Makula verhindern und somit den Schutz des Auges vor der Entstehung einer altersbedingten Makuladegeneration verbessern kann.

In bevorzugten Ausführungsformen umfasst die Lutein-enthaltende Zusammensetzung, bezogen auf das Gesamtgewicht der Zusammensetzung, wobei die Zusammensetzung auf eine Tablette oder Tagesdosis bezogen ist:
a. 0,1 Gew.-% bis 4 Gew.-%, bevorzugt 0,5 Gew.-% bis 2 Gew.-%, besonders bevorzugt 0,9 Gew.-% bis 1 Gew.-%, Lutein;
b. 0,1 Gew.-% bis 6,5 Gew.-%, bevorzugt 0,7 Gew.-% bis 3,2 Gew.-%, besonders bevorzugt 1,5 Gew.-% bis 1,6 Gew.-%, Zink*¹;
c. 0,007 Gew.-% bis 0,2 Gew.-%, bevorzugt 0,018 Gew.-% bis 0,08 Gew.-%, besonders bevorzugt 0,035 Gew.-% bis 0,04 Gew.-%, Kupfer*¹;
d. 1,5 Gew.-% bis 40 Gew.-%, bevorzugt 4,5 Gew.-% bis 20 Gew.-%, besonders bevorzugt 9 Gew.-% bis 10 Gew.-%, Vitamin C;
e. 0,07 Gew.-% bis 20 Gew.-%, bevorzugt 2,2 Gew.-% bis 10 Gew.-%, besonders bevorzugt 4,5 Gew.-% bis 5 Gew.-%, Vitamin E;
f. 0,1 Gew.-% bis 6,5 Gew.-%, bevorzugt 0,7 Gew.-% bis 3,2 Gew.-%, besonders bevorzugt 1,5 Gew.-% bis 1,6 Gew.-%, Niacinamid.
*¹: Gewichtsgehalt Gew.-% ist auf das Metall bezogen, wobei Zink und Kupfer als Gluconat vorliegen.

Die Lutein-enthaltende Zusammensetzung weist Zink und Kupfer in Form ihrer Salze oder anderer geeigneter Verbindungen auf, vorzugsweise in Form von Zinkgluconat und Kupfer(II)-gluconat. Angaben der enthaltenen Gehalte an Zink und Kupfer in Gew.-% bzw. Milligramm sind bezogen auf den Gehalt an Zink und Kupfer und nicht auf den Gehalt an Zink- bzw. Kupfer(II)-gluconat oder anderen Zink- bzw. Kupferverbindungen. Hierbei sind die Gewichtsgehalte der jeweiligen Stoffe so gewählt, dass das Gesamtgewicht der Stoffe 100 Gew.-% nicht übersteigt.

In seiner Funktion als Nahrungsergänzungsmittel oder als Mittel zur ergänzenden bilanzierten Diät weist die Lutein-enthaltende Zusammensetzung Lutein auf, das in ausgewogener Kombination mit den Spurenelementen Zink und Kupfer und weiteren antioxidativ wirkenden Vitaminen eine ausgewogene Versorgung der Makula bereit stellen kann. Dies kann zu einer vorbeugenden Wirkung der Zusammensetzung in Bezug auf eine Schädigung der Makula durch Radikale beitragen, wodurch die Entstehung einer altersbedingten Makuladegeneration positiv beeinflusst und eine Verbesserung der Sehfähigkeit im Alter erreicht werden kann. Es hat sich weiterhin überraschend gezeigt, dass das synergistische Zusammenwirken des vitaminähnlichen Carotinoids Lutein mit Zink und Kupfer, vorzugsweise mit Zinkgluconat und Kupfer(II)-gluconat, sowie mit Vitamin C, Vitamin E und Niacinamid eine verbesserte antioxidative Wirkung der Zusammensetzung bewirken kann.

Die vorteilhaften Eigenschaften ergeben sich aus der optimierten Auswahl der einzelnen Stoffe, die speziell zur Bekämpfung einer oxidativen Schädigungen der Makula durch Radikale ausgewählt wurden, um ein Defizit an Antioxidantien ausgleichen zu können. Unter dem Begriff "Stoff" sind im Sinne dieser Erfindung die Stoffe zu verstehen, die die die Lutein-enthaltende Zusammensetzung aufweist, insbesondere Lutein, Zink, Kupfer, vorzugsweise Zinkgluconat und Kupfer(II)-gluconat, Vitamin C, Vitamin E und Niacinamid.

Die Lutein-enthaltende Zusammensetzung kann auch weitere Vitamine und/oder Provitamine enthalten. Verwendbar sind beispielsweise handelsübliche Formen der Stoffe, wobei die jeweils geeignete Form entsprechend der jeweiligen Darreichungsform verwendet werden kann.

In vorteilhaften Ausführungsformen umfasst die Lutein-enthaltende Zusammensetzung, bezogen auf eine Tagesdosis der Zusammensetzung:
a. 1 mg bis 24 mg, bevorzugt 3 mg bis 12 mg, Lutein;
b. 1 mg bis 40 mg, bevorzugt 5 mg bis 20 mg, Zink*¹;
c. 0,05 mg bis 1 mg, bevorzugt 0,12 mg bis 0,5 mg, Kupfer*¹;
d. 10 mg bis 240 mg, bevorzugt 30 mg bis 120 mg, Vitamin C;
e. 5 mg bis 120 mg, bevorzugt 15 mg bis 60 mg, Vitamin E;
f. 1 mg bis 40 mg, bevorzugt 5 mg bis 20 mg, Niacinamid.
*¹: Gewichtsgehalt in mg bezogen auf das Metall, wobei Zink und Kupfer als Gluconat vorliegen.

Vorteilhaft ist darüber hinaus, dass die Dosierungen der erfindungsgemäßen Zusammensetzung keine schädigenden Nebenwirkungen verursachen und zur Prophylaxe wie auch zum Ausgleich einer bestehenden unzureichenden Versorgung der Makula dienen können. Dies ist beispielsweise zur ergänzenden bilanzierten Diät wie auch zur diätetischen Behandlung oder Begleitung bei altersbedingter Makuladegeneration vorteilhaft. Ein positiver Effekt zeigt sich insbesondere durch eine ergänzende bilanzierte Diät oder diätetische Verabreichung bei Mangelzuständen und der Vorbeugung der Entstehung einer Mangelsituation, die zu einer altersbedingten Makuladegeneration führen kann.

Es können Ausführungsformen der Lutein-enthaltenden Zusammensetzung geeignet sein, die keine weiteren Carotinoide enthalten. Eine Verabreichung weiterer Carotinoide kann einen nachteiligen Effekt auf die Resorption haben und/oder eine mögliche Interaktion der Carotinoide bewirken. Es kann beispielsweise eine Ausführungsform der Lutein-enthaltenden Zusammensetzung erfindungsgemäß geeignet sein, die frei von Beta-Carotin und/oder Kupferoxid ist. Eine orale Einnahme von Beta-Carotin kann insbesondere bei Rauchern in hohen Dosierungen zu Schädigungen in Verbindung mit Arzneimitteln führen. Die erfindungsgemäße Lutein-enthaltende Zusammensetzung kann frei von Beta-Carotin sein. In vorteilhafter Weise wird durch eine Ausführungsform der Lutein-enthaltenden Zusammensetzung, die frei von Beta-Carotin ist, die Möglichkeit geschaffen, auch von Rauchern eingenommen werden zu können.

Die Lutein-enthaltende Zusammensetzung kann fest-, flüssig und/oder gelförmig vorliegen, vorzugsweise liegt die Zusammensetzung in Darreichungsformen, ausgewählt aus der Gruppe umfassend Tabletten, Filmtabletten, Dragees, Kapseln, Pulver, Granulat, Lösungen und/oder Brausetabletten, vor. Die Zusammensetzung kann in ein oder mehreren, vorzugsweise ein bis drei, Darreichungsformen ausgewählt aus der Gruppe umfassend Tabletten, Filmtabletten, Dragees, Kapseln, Pulver, Granulat, Lösungen und/oder Brausetabletten vorliegen, wobei die Darreichungsformen gleiche oder unterschiedliche, vorzugsweise gleiche Zusammensetzungen an Stoffen aufweisen können.

Bevorzugt liegt die Lutein-enthaltende Zusammensetzung, insbesondere als Nahrungsergänzungsmittel, in Form einer Tablette, insbesondere einer Filmtablette vor. Vorzugsweise umfasst die Tablette einen Tablettenkern, der mit einem Filmüberzug beschichtet sein kann. Bevorzugt umfasst die Tablette einen Tablettenkern, der mit einem Filmüberzug versehen ist.

In bevorzugten Ausführungsformen liegt die Lutein-enthaltende Zusammensetzung in Form einer festförmigen Darreichungsform vor, wobei der Kern der Darreichungsform bevorzugt 200 mg bis 1000 mg, vorzugsweise 400 mg bis 800 mg, und besonders bevorzugt 600 mg bis 650 mg, ganz besonders bevorzugt 620 mg ausmacht. Der Kern der Darreichungsform ist vorzugsweise mit einem Filmüberzug beschichtet.

Die aufgetragene Filmmenge beträgt vorzugsweise 1 Gew.-% bis 12 Gew.-%, bevorzugt 2 Gew.-% bis 10 Gew.-%, besonders bevorzugt 3 Gew.-% bis 8 Gew.-%, bezogen auf das Gewicht des Kerns. Ganz besonders bevorzugt beträgt die aufgetragene Filmmenge 40 mg.

In bevorzugten Ausführungsformen einer Filmtablette beträgt das Gewicht der Filmtablette 660 mg, der Durchmesser liegt bei < 11,9 mm und/oder die Dicke bei < 6,0 mm, wobei das Gewicht des Tablettenkerns 620 mg ausmacht, der Durchmesser des Tablettenkerns bei < 11,7 mm, die Dicke des Tablettenkerns im Bereich von 5,6 mm bis 5,8 mm und/oder die Härte im Bereich von 65 kN bis 95 kN liegt.

Die Lutein-enthaltende Zusammensetzung, insbesondere der Tablettenkern, weist gegebenenfalls weitere Hilfsstoffe auf. Diese Hilfsstoffe sind vorzugsweise ausgewählt aus der Gruppe umfassend Lactose, Povidon, Cellulose und/oder Cellulosederivate, mikrokristalline Cellulose, Stärke und/oder Stärkederivate, Magnesiumstearat, Stearinsäure, Gelatine, Natriumaluminiumsilikat, Siliciumdioxid, Maltodextrin, Dextrose, Talkum, Titandioxid, Calciumcarbonat, Pflanzenfette, Tricalciumphosphat, Antioxidantien, Stabilisatoren, Gummi arabicum, Calciumphosphate, Calciumsilikat, Saccharose, Natriumcitrat und/oder Zitronensäure. Besonders bevorzugte Hilfsstoffe sind ausgewählt aus der Gruppe umfassend mikrokristalline Cellulose und/oder Magnesiumstearat.

Weiterhin kann die Lutein-enthaltende Zusammensetzung Überzugsmittel, auch bezeichnet als Coatingstoffe, aufweisen. Beispielsweise kann die Lutein-enthaltende Zusammensetzung, insbesondere in Form einer Filmtablette, geeignete Coatingstoffe ausgewählt aus der Gruppe umfassend Cellulose und/oder Cellulosederivate, Titandioxid, Farbstoffe, bevorzugt Eisenoxide und/oder Chinolinegelb Al-Lack, Hydroxypropylcellulose, Hydroxypropylmethylcellulose, Talkum, Miglyol 812, Polymethacrylate, Schellack, Polyvinylpyrolidon, Polyvinylalkohol, Polyethylenglykole, Triacetin, Triethylcitrat, Propylenglykol, Glycerol, Gummi arabicum, Siliciumdioxid, Glycerolmonostearat aufweisen. Bevorzugt sind Coatingstoffe ausgewählt aus der Gruppe umfassend Hydroxypropylcellulose, Hydroxypropylmethylcellulose, Talkum, Miglyol 812, Titandioxid und/oder Farbstoffe, bevorzugt Eisenoxide und/oder Chinolinegelb Al-Lack.

Die Herstellung der Darreichungsformen, beispielsweise Tablette, Dragee oder Kapsel, erfolgt vorzugsweise nach allgemein üblichen Verfahren, beispielsweise durch Kompaktierung, Granulierung und Abfüllung in Gelatinekapseln, etc.. Üblicherweise werden zur Herstellung von Tabletten die Tablettenkernbestandteile trocken vermischt und zu Tabletten verpresst. Anschließend wird beispielsweise durch Sprühen oder Lackieren der Filmüberzug aus einer geeigneten Lösung oder Suspension der Coatingbestandteile aufgetragen.

Die Stoffe werden bevorzugt in einer für die gewählte Darreichungsform verarbeitbaren Form eingesetzt. Bevorzugt weist die Lutein-enthaltende Zusammensetzung Vitamin E in Form von Vitamin E-acetat auf. Bevorzugt weist die Zusammensetzung Vitamin C in Form eines Granulats auf. Weiterhin bevorzugt weist die Lutein-enthaltende Zusammensetzung Vitamin C in Form von Ascorbinsäure auf. In besonders bevorzugten Ausführungsformen weist die Lutein-enthaltende Zusammensetzung Lutein, Zinkgluconat, Kupfer(II)-gluconat, Vitamin E-acetat, Ascorbinsäure, Niacinamid, mikrokristalline Cellulose und/oder Magnesiumstearat auf.

In einer bevorzugten Ausführungsformen umfasst die Lutein-enthaltende Zusammensetzung, bezogen auf eine Tablette oder Tagesdosis der Zusammensetzung:
a. 6 mg Lutein;
b. 10 mg Zink*¹;
c. 0,25 mg Kupfer*¹;
d. 60 mg Vitamin C;
e. 30 mg Vitamin E;
f. 10 mg Niacinamid.
^{*1}: Gewichtsgehalt in mg bezogen auf das Metall, wobei Zink und Kupfer als Gluconat vorliegen.

In einer bevorzugten Ausführungsformen umfasst die Lutein-enthaltende Zusammensetzung, bezogen auf eine Einzeldosis der Zusammensetzung:
a. 6 mg Lutein;
b. 10 mg Zink*¹;
c. 0,25 mg Kupfer*¹;
d. 60 mg Vitamin C;
e. 30 mg Vitamin E;
f. 10 mg Niacinamid.
^{*1}: Gewichtsgehalt in mg bezogen auf das Metall, wobei Zink und Kupfer als Gluconat vorliegen.

Unter einer Einzeldosis wird im Sinne dieser Anmeldung eine Verabreichungseinheit der Lutein-enthaltenden Zusammensetzung verstanden, eine Einzeldosis der Lutein-enthaltenden Zusammensetzung entspricht beispielsweise einer Tablette, einer Filmtablette, einem Dragee, einer Kapsel oder einer einzelnen Verabreichungseinheit einer anderen Darreichungsform, wie Pulver oder Granulat. Unter einer Tagesdosis wird im Sinne dieser Anmeldung die Menge der Zusammensetzung verstanden, die pro Tag verabreicht wird.

Die Tagesdosis und/oder Einzeldosis ist vorzugsweise einmal oder auf mehrere gleiche oder unterschiedliche Darreichungsformen verteilt, wobei die Darreichungsformen gleiche oder unterschiedliche Stoffe und/oder Stoffgewichtsgehalte aufweisen können. Vorzugsweise entspricht die Tagesdosis einer Einzeldosis.

Die Zusammensetzung kann auch Zusätze, Begleitstoffe und/oder Rohstoffe enthalten, die schwerer sind als die eigentlichen Wirkstoffe, so dass die angegebenen Mengen bezogen auf eine Tagesdosis bzw. Einzeldosis niedriger oder auch höher sein können.

Die Lutein-enthaltende Zusammensetzung kann zur Herstellung eines Nahrungsergänzungsmittels oder als Mittel zur diätetischen Begleitung, beispielsweise bei Augenkrankheiten, bevorzugt bei altersbedingter Makuladegeneration, verwendet werden. Die Lutein-enthaltende Zusammensetzung ist weiterhin geeignet als Nahrungsergänzungsmittel oder als Mittel zur ergänzenden bilanzierten Diät.

Der Begriff "diätetische Behandlung" ist in der EU-Richtlinie 1999/21/EG, die seit 01.01.2002 als 10. Verordnung zur Änderung der Diätverordnung in deutsches Recht umgesetzt worden ist, näher erläutert.

Durch die vorteilhaften Stoffe der Lutein-enthaltende Zusammensetzung kann diese auch im Rahmen einer diätetischen Ernährungsberatung als Nahrungsergänzungsmittel oder als Mittel zur ergänzenden bilanzierten Diät, insbesondere Lebensmittel, eingesetzt werden. Bevorzugt kann die Lutein-enthaltende Zusammensetzung zur ergänzenden bilanzierten Diät bei altersbedingter Makuladegeneration eingesetzt werden. Es hat sich gezeigt, dass insbesondere ein möglicher Nährstoffmangel bei älteren Menschen ausgeglichen werden kann.

Beispiele für Lutein-enthaltende Zusammensetzungen sind nachstehend angegeben:

Es versteht sich, dass die Darreichungsformen, ausgewählt aus der Gruppe umfassend Tabletten, Filmtabletten, Dragees, Kapseln, Pulver, Granulat, Lösungen und/oder Brausetabletten, die üblichen zur Formulierung der jeweiligen Darreichungsformen eingesetzten Hilfsstoffe aufweisen, so dass in den Beispielen lediglich die enthaltenen Wirkstoffe aufgeführt sein können.

### Beispiel 1

Lutein-enthaltende Zusammensetzung, Tagesdosis umfassend 1 Filmtablette mit folgenden Stoffen:
- 6 mg Lutein;
- 10 mg Zink^{*1};
- 0,25 mg Kupfer^{*1};
- 60 mg Vitamin C;
- 30 mg Vitamin E;
- 10 mg Niacinamid.
*¹: Gewichtsgehalt in mg bezogen auf das Metall, wobei Zink und Kupfer als Gluconat vorliegen.

### Beispiel 2

Rezeptur für 1 Tablettenkern ä 620 mg, umfassend:
- 144 mg Lutein, 5% TG^{*3};
- 75,79 mg Ascorbinsäure, Granulat, 95 %^{*3};
- 107,28 mg Dry Vitamin E Acetat, 50 % DC^{*3};
- 76,643 mg Zinkgluconat^{*3};
- 1,97 mg Kupfer(II)-gluconat^{*3};
- 11 mg Niacinamid^{*3};
- 197,117 mg Cellulose, mikrokristallin;
- 6,2 mg Magnesiumstearat.
*³: In den angegebenen Mengen sind weitere übliche Hilfsstoffe, wie Stabilisatoren und/oder Stabilitätszuschläge und/oder Produktionszuschläge enthalten.

Rezeptur für 1 Filmüberzug, umfassend:
- 40 mg Coating-Material^{*2};
- 320 mg gereinigtes Wasser.
^{*2}: Coating-Material auf Basis von Hydroxypropylcellulose, Hydroxypropylmethylcellulose, Talkum, Miglyol 812, Titandioxid und Farbstoffe, bevorzugt Eisenoxide und/oder Chinolinegelb Al-Lack.

### Beispiel 3

Rezeptur für 403,001 kg, umfassend pro 650.000 Tabletten:
- 93,6 kg Lutein, 5% TG^{*3};
- 49,264 kg Ascorbinsäure, Granulat, 95 %^{*3};
- 69,732 kg Dry Vitamin E Acetat, 50 % DC^{*3};
- 49,818 kg Zinkgluconat^{*3};
- 1,281 kg Kupfer(II)-gluconat^{*3};
- 7,15 kg Niacinamid^{*3};
- 128,126 kg mg Cellulose, mikrokristallin;
- 4,030 kg Magnesiumstearat.
^{*3}: In den angegebenen Mengen sind weitere übliche Hilfsstoffe, wie Stabilisatoren und/oder Stabilitätszuschläge und/oder Produktionszuschläge enthalten.

Rezeptur für 1 Filmüberzug, umfassend:
- 26 kg Coating-Material^{*2};
- 208 kg gereinigtes Wasser.
^{*2}: Coating-Material auf Basis von Hydroxypropylcellulose, Hydroxypropylmethylcellulose, Talkum, Miglyol 812, Titandioxid und Farbstoffe, bevorzugt Eisenoxide und/oder Chinolinegelb Al-Lack.

## Patentansprüche

1. Lutein-enthaltende Zusammensetzung, **dadurch gekennzeichnet, dass** die Zusammensetzung Lutein sowie Zink- und Kupferverbindungen, vorzugsweise in Form von Zinkgluconat und Kupfer(II)-gluconat, umfasst.

2. Lutein-enthaltende Zusammensetzung nach Anspruch 1, **dadurch gekennzeichnet, dass** die Zusammensetzung wenigstens ein Vitamin, vorzugsweise ausgewählt aus der Gruppe umfassend Vitamin C, Vitamin E und/oder Niacinamid, aufweist.

3. Lutein-enthaltende Zusammensetzung nach Anspruch 1 oder 2, **dadurch gekennzeichnet, dass** die Zusammensetzung, bezogen auf das Gesamtgewicht der Zusammensetzung, umfasst:
a. 0,1 Gew.-% bis 4 Gew.-%, bevorzugt 0,5 Gew.-% bis 2 Gew.-%, besonders bevorzugt 0,9 Gew.-% bis 1 Gew.-%, Lutein;
b. 0,1 Gew.-% bis 6,5 Gew.-%, bevorzugt 0,7 Gew.-% bis 3,2 Gew.-%, besonders bevorzugt 1,5 Gew.-% bis 1,6 Gew.-%, Zink;
c. 0,007 Gew.-% bis 0,2 Gew.-%, bevorzugt 0,018 Gew.-% bis 0,08 Gew.-%, besonders bevorzugt 0,035 Gew.-% bis 0,04 Gew.-%, Kupfer;
d. 1,5 Gew.-% bis 40 Gew.-%, bevorzugt 4,5 Gew.-% bis 20 Gew.-%, besonders bevorzugt 9 Gew.-% bis 10 Gew.-%, Vitamin C;
e. 0,07 Gew.-% bis 20 Gew.-%, bevorzugt 2,2 Gew.-% bis 10 Gew.-%, besonders bevorzugt 4,5 Gew.-% bis 5 Gew.-%, Vitamin E;
f. 0,1 Gew.-% bis 6,5 Gew.-%, bevorzugt 0,7 Gew.-% bis 3,2 Gew.-%, besonders bevorzugt 1,5 Gew.-% bis 1,6 Gew.-%, Niacinamid.

4. Lutein-enthaltende Zusammensetzung nach einem der vorherigen Ansprüche, **dadurch gekennzeichnet, dass** die Zusammensetzung, bezogen auf eine Tagesdosis der Zusammensetzung, umfasst:
a. 1 mg bis 24 mg, bevorzugt 3 mg bis 12 mg, Lutein;
b. 1 mg bis 40 mg, bevorzugt 5 mg bis 20 mg, Zink;
c. 0,05 mg bis 1 mg, bevorzugt 0,12 mg bis 0,5 mg, Kupfer;
d. 10 mg bis 240 mg, bevorzugt 30 mg bis 120 mg, Vitamin C;
e. 5 mg bis 120 mg, bevorzugt 15 mg bis 60 mg, Vitamin E;
f. 1 mg bis 40 mg, bevorzugt 5 mg bis 20 mg, Niacinamid.

5. Lutein-enthaltende Zusammensetzung nach einem der vorherigen Ansprüche, **dadurch gekennzeichnet, dass** die Zusammensetzung fest-, flüssig und/oder gelförmig vorliegt, vorzugsweise liegt die Zusammensetzung in Darreichungsformen, ausgewählt aus der Gruppe umfassend Tabletten, Filmtabletten, Dragees, Kapseln, Pulver, Granulat, Lösungen und/oder Brausetabletten, vor.

6. Lutein-enthaltende Zusammensetzung nach einem der vorherigen Ansprüche, **dadurch gekennzeichnet, dass** die Zusammensetzung in einer sogenannten festen Darreichungsform vorliegt, wobei der Kern der Darreichungsform 200 mg bis 1000 mg, vorzugsweise 400 mg bis 800 mg, bevorzugt 600 mg bis 650 mg und besonders bevorzugt 620 mg ausmacht.

7. Lutein-enthaltende Zusammensetzung nach einem der vorherigen Ansprüche, **dadurch gekennzeichnet, dass** die Zusammensetzung Hilfsstoffe ausgewählt aus der Gruppe umfassend Cellulose, vorzugsweise mikrokristalline Cellulose, und/oder Magnesiumstearat aufweist.

8. Lutein-enthaltende Zusammensetzung nach einem der vorherigen Ansprüche, **dadurch gekennzeichnet, dass** die Zusammensetzung Lutein, Zinkgluconat, Kupfer(II)-gluconat, Vitamin E-acetat, Ascorbinsäure, Niacinamid, mikrokristalline Cellulose und/oder Magnesiumstearat aufweist.

9. Lutein-enthaltende Zusammensetzung nach einem der vorherigen Ansprüche, **dadurch gekennzeichnet, dass** die Zusammensetzung, bezogen auf eine Tagesdosis der Zusammensetzung, umfasst:
a. 6 mg Lutein;
b. 10 mg Zink;
c. 0,25 mg Kupfer;
d. 60 mg Vitamin C;
e. 30 mg Vitamin E;
f. 10 mg Niacinamid.

10. Lutein-enthaltende Zusammensetzung nach einem der vorherigen Ansprüche, **dadurch gekennzeichnet, dass** die Zusammensetzung, bezogen auf eine Einzeldosis der Zusammensetzung, umfasst:
a. 6 mg Lutein;
b. 10 mg Zink;
c. 0,25 mg Kupfer;
d. 60 mg Vitamin C;
e. 30 mg Vitamin E;
f. 10 mg Niacinamid.

11. Verwendung einer Lutein-enthaltenden Zusammensetzung nach einem der vorherigen Ansprüche zur Herstellung eines Mittels zur diätetischen Verhütung und/oder Behandlung von Augenkrankheiten, bevorzugt bei altersbedingter Makuladegeneration.

12. Verwendung einer Lutein-enthaltenden Zusammensetzung nach einem der vorherigen Ansprüche als Nahrungsergänzungsmittel und/oder Mittel zur ergänzenden bilanzierten Diät.

13. Mittel, Nahrungsergänzungsmittel oder Mittel zur ergänzenden bilanzierten Diät, insbesondere Lebensmittel, umfassend Stoffe gemäß einem der vorherigen Ansprüche.
